# EUROPÄISCHE PATENTSCHRIFT

(11) **EP 0 910 993 B1**
(45) Veröffentlichungstag und Bekanntmachung des Hinweises auf die Patenterteilung: **17.07.2002**
(21) Anmeldenummer: 98119329.5
(22) Anmeldetag: 13.10.1998
(51) Int. Cl.: A61B 17/80

(54) **Gitter für die Fixierung von Knochenteilen oder für die Überbrückung von Knochenfehlstellen**
Mesh for fixing bone fragments or for bridging bone defects
Grille pour la fixation de fragments d'os ou pour traverser un défaut osseux

(30) Priorität: 21.10.1997 DE 19746396
(43) Veröffentlichungstag der Anmeldung: 28.04.1999
(73) Patentinhaber: Stryker Leibinger GmbH & Co. KG, 79111 Freiburg (DE)
(72) Erfinder: Reisberg, Erhard, 79219 Stauben (DE); Forst, Peter, D-79312 Emmendingen (DE)
(74) Vertreter: von Hellfeld, Axel, Dr. Dipl.-Phys.

(56) Entgegenhaltungen:
- DE-A- 19 507 544
- US-A- 5 468 242
- US-A- 5 509 933
- US-A- 5 743 913

## Beschreibung

Die Erfindung betrifft ein Gitter für die Fixierung von Knochenteilen oder für die Überbrückung von Knochenfehlstellen, insbesondere im Schädel- und Kieferbereich, aus biokompatiblem Material mit einer netzartigen Struktur.

Ein solches Gitter ist aus der US-A-5,468,242 bekannt. Derartige formbare Gitter-Implantate dienen der Fixierung und Immobilisierung von Knochenfragmenten an einer oder mehreren Knochenbruchstellen und insbesondere auch als dreidimensional formbares Material, welches mittels Knochenschrauben am Knochen befestigt wird. Mit solchen Gittern können nicht nur flache Knochenbereiche abgedeckt werden, sondern auch gekrümmte Knochenoberflächen, einschließlich konkaver, konvexer oder sphärischer Oberflächen, bei denen verschiedene Bereiche des Gitters unterschiedlich stark gedehnt werden müssen, während andere Bereiche des Gitters nicht gedehnt werden müssen oder in seltenen Fällen sogar gestaucht werden müssen. Bei dem zitierten Stand der Technik dienen kreisförmige Ringe, die regelmäßig im Netz verteilt sind, zur Aufnahme der Knochenschrauben. Diese Ringe schränken die Dehn- und Formbarkeit des Gitters in gewissem Umfang ein. Zwischen den Aufnahmen für die Knochenschrauben verlaufen bei diesem Stand der Technik Stege. Diese Stege des Gitters bilden keine Kreuzungspunkte, vielmehr liegen die Stege (dort als Arme bezeichnet) zwischen den ringförmigen Aufnahmen für die Knochenschrauben, welche jeweils an den Netz-Kreuzungspunkten des Gitters liegen. Jene Gitter dienen hauptsächlich zur Defekt-Überbrückung in nicht lasttragenden Knochenbereichen.

Die EP 0 566 255 A1 beschreibt ein Gitter mit ungekrümmten Stegen. Dort wird der Vorteil der geradlinigen, quadratischen Stegformen besonders betont. Auch die DE 195 07 544 A1 beschreibt ein Gitter für die Chirurgie. Das Gitter hat dort eine sechsekkige Grundstruktur. Es zeigt keine Stege, die zwischen den Gitter- Kreuzungspunkten gekrümmt sind.

Der Erfindung liegt die Aufgabe zugrunde, das eingangs genannte, dreidimensional formbare Gitter (Fachausdruck "Mesh") so weiterzubilden, daß eine noch weiter verbesserte dreidimensionale Biegbarkeit gegeben ist. Dabei sollen insbesondere auch die Perforationen des Gitters selbst bei maximaler Inanspruchnahme bei einer Erweiterung und/oder Dehnung des Gitters jedoch klein genug bleiben, um Weichteile am Durchwachsen und damit Eindringen in Kavitäten zu hindern und andererseits soll jedoch auch der Flüssigkeitsaustausch durch das Gitter nicht behindert werden.

Erfindungsgemäß wird dieses Ziel dadurch erreicht, daß das Gitter der eingangs genannten Art eine Vielzahl von Gitterpunkten aufweist, an denen gekrümmte Gitter-Stege enden.

Unter einem Gitterpunkt soll hier ein Punkt (eine Stelle) des ebenen Gitters verstanden werden, an dem mehrere, mindestens aber drei Stege des Gitters enden. Mit anderen Worten: An dem Gitterpunkt laufen mindestens drei Stege zusammen. Der Begriff "Punkt" ist hier nicht mathematisch, sondern technisch im Sinne eines bestimmten Bereichs des Gitters zu verstehen. Entsprechend der vorstehenden Definition eines Gitterpunktes wird hier unter einem Steg die materielle Verbindung benachbarter Gitterpunkte verstanden, die sich zwischen ihren Enden weder teilt noch verzweigt, jedoch gerade oder in der Gitterebene gebogen sein kann. Enden an einem Gitterpunkt vier Stege, wird dieser hier auch als Kreuzungspunkt bezeichnet. Wenn im Stand der Technik die Stege in einem Ring enden, der zur Aufnahme einer Knochenschrauben dient, handelt es sich dabei nicht um einen Gitterpunkt, da bei solchem Stand der Technik die Stege nicht im Gitterpunkt enden, d.h. nicht im Gitterpunkt zusammen laufen.

Eine Folge aneinanderhängender Stege, die zusammen die kürzestmögliche durchgehende Linie in Richtung einer Gitterhauptachse bilden, wird hier als Stegreihe bezeichnet (eine Stegreihe ist bei den Ausführungsbeispielen der Erfindung keine gerade Linie). Weiterhin sind nachfolgend mit "Hauptachsen des Gitters" die Richtung derjenigen geraden Linien bezeichnet, die diejenigen benachbarten Gitterpunkte eines gegebenen Gitterpunktes verbinden, die von allen möglichen Gitterpunktpaaren die größte Entfernung voneinander haben. Dies ist in Fig. 4 dargestellt. Von einem Gitterpunkt ausgehend fügen sich also die Stege zu mäanderförmigen Linien zusammen, wobei die Mäanderlinien in Richtung der Hauptachsen laufen.

Gemäß einer besonderen Ausführung der Erfindung ist ein Gitter für die Fixierung von Knochenteilen oder für die Überbrückung von Knochenfehlstellen der eingangs genannten Art vorgesehen, bei dem das Gitter keine kreisringförmigen Aufnahmen für Knochenschrauben aufweist, jedoch jede Gitter-Ausnehmung zur Aufnahme einer Knochenschraube ausgebildet ist und die Stegreihen des Gitters, eine durchgehende periodische Wellenlinie bilden, wobei die Wellenlinie bevorzugt eine relativ weiche (runde), etwa sinusförmige Struktur hat.

Ein derartiges Gitter ermöglicht eine hohe dreidimensionale Anbiegbarkeit bei hoher Stabilität. Dabei können die Abmessungen der Ausnehmungen im Gitter so gering gehalten werden, daß das Gitter während des Heilungsprozesses nicht von Gewebe durchwachsen wird, wobei jedoch gleichzeitig ein weitestgehend ungehinderter Flüssigkeitsaustausch durch das Gewebe hindurch ermöglicht ist. Für die Plazierungen der Knochenschrauben hat der Operateur eine größere Wahlmöglichkeit als beim oben zitierten Stand der Technik.

Eine wichtige Eigenschaft des erfindungsgemäßen Gitters ist, daß es dreidimensional form- und dehnbar ist. Es ist also praktisch in allen Richtungen dehnbar und auch stauchbar, je nach Anforderung des Patienten.

Gemäß einer bevorzugten Ausgestaltung der Erfindung, nachfolgend "Mesh 1" genannt, ist vorgesehen, daß alle Stege zwischen allen Gitterpunkten des Gitters in zumindest zwei entgegengesetzte Richtungen gekrümmt sind.

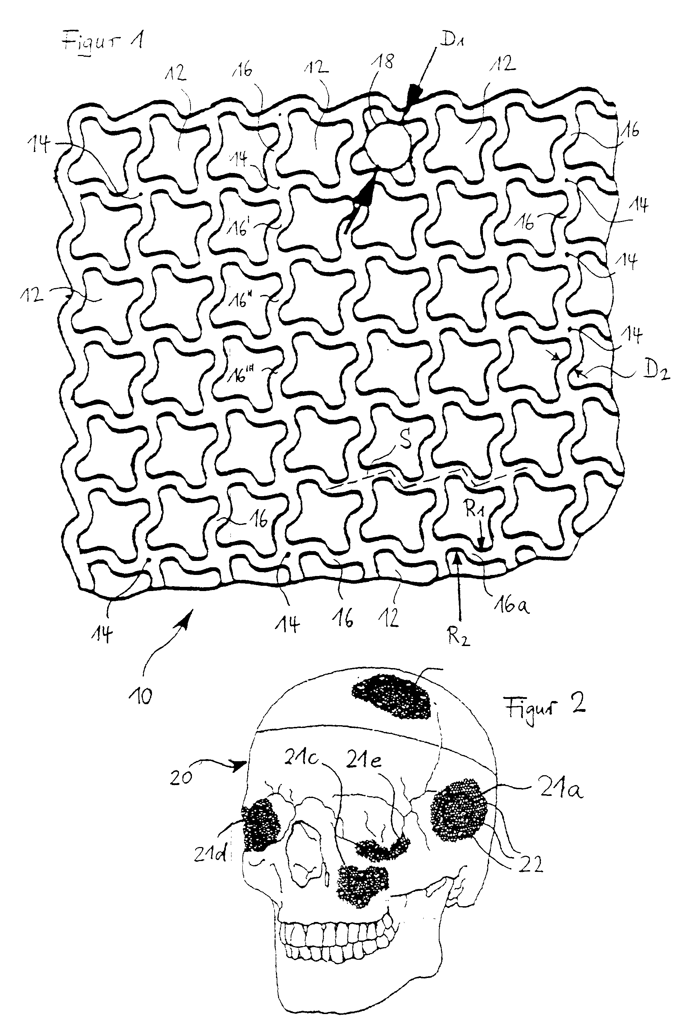

Beim Ausführungsbeispiel der Erfindung gemäß Fig.1 (Mesh 1) bilden die an eine Ausnehmung des Gitters angrenzenden Gitterpunkte ein Rechteck, insbesondere ein Quadrat. Jeder Gitterpunkt ist mit seinen vier benachbarten Gitterpunkten über jeweils einen Steg verbunden.

Eine weitere, besonders bevorzugte Variante der Erfindung, nachfolgend "Mesh 2" genannt, erreicht die vorstehend genannten technischen Ziele, insbesonder eine gegenüber Mesh 1 noch erhöhte Dehnungsfähigkeit und Rißfestigkeit, dadurch, daß die Gitterpunkte an den Ecken eines Sechseck-Wabenmusters angeordnet und über Stege verbunden sind, die nur in einer Richtung gekrümmt, vorzugsweise als Halbkreise ausgebildet sind. Bei dieser Variante der Erfindung sind die sechs an eine Ausnehmung angrenzenden Stege vom Mittelpunkt der Ausnehmung gesehen abwechselnd nach innen und nach außen gekrümmt.

Nachfolgend werden Ausführungsbeispiele der Erfindung anhand der Zeichnung näher erläutert. Es zeigt:
- Fig. 1: ein erfindungsgemäßes Gitter in stark vergrößertem Maßstab
- Fig. 2: beispielhaft Applikationen eines Gitters gemäß den Fig. 1 oder 3 am menschlichen Schädel bzw. Kiefer,
- Fig. 3: ein weiteres Ausführungsbeispiel eines bevorzugten erfindungsgemäßen Gitters in stark vergrößertem Maßstab, und
- Fig. 4: die Definition der Gitter-Hauptachsen bei einem Gitter gemäß Fig. 3

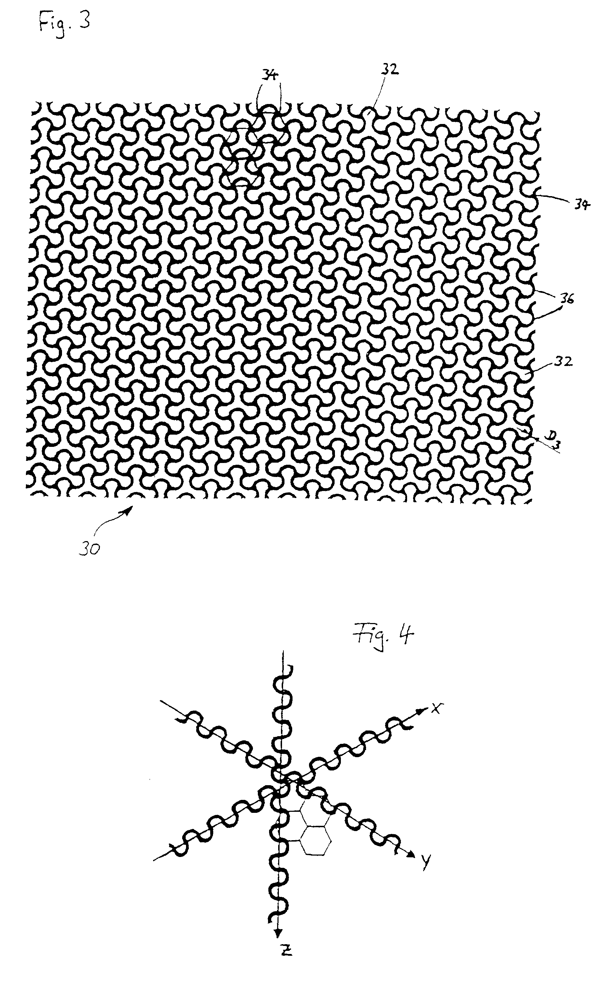

Die Figuren 1 und 3 zeigen Gitter für die Fixierung von Knochenteilen oder für die Überbrückung von Knochenfehlstellen, insbesondere zur Rekonstruktion von Defekten der Schädeldecke, der Orbita und der Nasennebenhöhlenwände sowie für die Rekonstruktionen im Kieferbereich und auch Kieferkamm-Augmentationen.

Die Stärke des Gitters 10 bzw. 30 senkrecht zur Zeichnungsebene gemäß den Figuren 1 und 3 liegt unter 0,5 mm, weiter bevorzugt unter 0,3 mm, besonders bevorzugt unter 0,2 mm und ganz besonders bevorzugt unter 0,15 mm.

Die Breite D₂ bzw. D₃ jedes Steges zwischen den Gitterpunkten 14 bzw. 34 liegt bevorzugt unter dem 2-fachen und weiter bevorzugt unter dem 1,5-fachen Stärke des Gitters senkrecht zur Zeichnungsebene.

Gitter gemäß den vorstehend genannten Dimensionen und gemäß den in den Figuren 1 bzw. 3 gezeigten Strukturen lassen sich bevorzugt aus Titan ätzen.

Das Gitter 10 ist in Fig. 1 gegenüber seinen tatsächlichen Abmessungen etwa 16-fach vergrößert und nur in einem Ausschnitt dargestellt, d.h. der rechte und der untere Rand des Gitters sind abgeschnitten, zum Beispiel mit einer geeigneten Schere.

Gitter 10 bzw. 30 sind in Fig. 2 schematisch in unterschiedlichen Applikationen an einem menschlichen Schädel gezeigt, in Form verschieden zurechtgeschnittener Gitter 21a, 21b, 21c, 21d und 21e, die für die Fixierung unterschiedlicher Knochenteile bzw. für die Überbrückung von Knochenfehlstellen dreidimensional angebogen und mittels Knochenschrauben 22 am Knochen befestigt sind. Insoweit entspricht die Anwendung der erfindungsgemäßen Gitter dem eingangs diskutierten Stand der Technik.

Das Gitter 10 weist gemäß Fig. 1 eine Vielzahl gleichförmiger Ausnehmungen 12 auf, in die wahlweise Knochenschrauben 22 einsetzbar sind. Stege 16 zwischen Gitterpunkten 14 des netzartigen Gitters 10 sind jeweils S-förmig gekrümmt (in die entgegengesetzten Richtungen R₁, R₂). Diese Krümmung ermöglicht eine leichte Anbiegung des Gitters entsprechend den Erfordernissen des Knochendefektes oder der Knochenfehlstelle. Wie Fig. 1 zeigt, weist das Gitter 10 keine gesonderten kreisringförmigen Aufnahmen für die Knochenschrauben auf. Vielmehr können die Knochenschrauben durch eine beliebige Ausnehmung 12 geschoben werden, wobei die in Fig. 1 erkennbare kleeblattförmige Gestalt der Ausnehmungen 12 gute Auflageflächen des Knochenschraubenkopfes auf dem Gitter 10 liefert, nämlich auf vier jeweils um 90° getrennten Vorsprüngen der Stege 16 des Gitters.

Wie Fig. 1 weiter zeigt, bilden in einer Richtung ineinander übergehenden Stegen 16, 16,', 16'', 16''' etc. eine durchgehende periodische Wellenlinie, in annähernd abgerundeter Sägezahnstruktur. Diese Sägezahnstruktur ist in Fig. 1 mit einer gestrichelten Linie S angedeutet.

In einer Ausnehmung 12 kann jeweils ein Kreis 18 beschrieben werden, dessen Durchmesser D₁ bevorzugt kleiner als 2 mm, weiter bevorzugt kleiner als 1 mm und besonders bevorzugt kleiner als 0,8 mm ist. Der Kreis berührt jeweils die in die Ausnehmung 12 ragenden Krümmungen der Stege 16. Entsprechend überlappt ein Schraubenkopf (nicht gezeigt), der im Durchmesser etwas größer ist als der Kreis 18, die Stege 16 an diesen Krümmungsstellen, und zwar unter regelmäßigen Abständen von jeweils 90° über seinem Umfang, so daß ein gleichmäßiges Andrücken und Greifen durch den Schraubenkopf gewährleistet ist.

Fig. 3 zeigt eine weitere Ausführungsform eines Gitters, welches gegenüber dem vorstehend beschriebenen Gitter insbesondere auch hinsichtlich der Rißfestigkeit und der Dehnfähigkeit verbessert ist. Ansonsten hat das Gitter gemäß Fig. 3 auch noch bessere Eigenschaften mit Blick auf die übrigen, in der Beschreibungseinleitung genannten technischen und medizinischen Zielsetzungen.

Ein Gitter 30 gemäß Fig. 3 kann beschrieben werden als eine Struktur mit mäanderförmigen, durchgehenden, periodischen Stegreihen entlang der Hauptachsen x, y, z des Gitters (vgl. die obige Definition), jeweils ausgehend von einem Gitterpunkt. Fig. 4 zeigt die Definition der Hauptachsen x, y, z des Gitters.

Die kreisbogenförmigen Biegungen der Stege bilden jeweils Ausnehmungen (Perforationen) 32 im Gitter, wobei die Ausnehmungen 32 jeweils zur Aufnahme einer (einzigen) Knochenschraube geeignet sind. Die Ausnehmungen 32 sind nicht von einem vollständigen Steg-Ring umgeben, vielmehr bilden die Stege nur einen unvollständigen Ring, d.h. einen Ring, der größer ist als ein halbkreisförmiger Ring, aber kleiner als ein um 360° geschlossener Ring. Wie sich aus Fig. 3 unmittelbar ergibt, hat die dort gezeigte Struktur den besonderen Vorteil, daß für den Chirurgen eine sehr große Wahlfreiheit hinsichtlich der Positionierung von Knochenschrauben ermöglicht ist, d.h. es können Knochenschrauben praktisch an jeder Stelle des Gitters gesetzt werden. Die Aufnahmen 32 für die Knochenschrauben sind dabei im wesentlichen gleichwertig.

Die in Fig. 3 gezeigte Struktur eines Gitters kann auch mittels der Gitterpunkte 34 beschrieben werden. Wie aus Fig. 3 zu erkennen ist, gehen von jedem Gitterpunkt 34 drei Stege ab. Verbindet man einander benachbarte Gitterpunkte, dann bilden sich regelmäßige Sechsecke. In Fig. 3 sind oben rechts beispielhaft drei Sechsecke eingezeichnet (die Sechseck-Linien sind eine gezeichnete Ergänzung des Gitters, beim tatsächlichen Gitter aber nicht vorhanden). Die durch die (gedachten bzw. gezeichneten) Verbindungslinien der Gitterpunkte 34 gebildeten regelmäßigen Sechsekke bilden eine wabenförmige Struktur. Zwischen den Gitterpunkten 34 verlaufende Stege sind jeweils halbkreisförmig ausgestaltet und wölben sich abwechselnd einmal nach außen und zum anderen nach innen.

Beim Ausführungsbeispiel gemäß Fig. 3 ist die gesamte Gitterstruktur durch rund gekrümmte Stege gekennzeichnet, d.h. die Stege haben bei diesem Ausführungsbeispiel keine scharfen Knickstellen. Das in Fig. 3 gezeigte Gitter ist gegenüber dem tatsächlichen Gitter stark vergrößert dargestellt, etwa im Maßstab 1:10.

Die Kantenlänge des Sechsecks, also die Länge einer Verbindungslinie der Gitterpunkte beträgt zum Beispiel das 4 bis 8-fache der Blechdicke des Gitters. Die Blechdicke des Gitters ist die Stärke des Gitters senkrecht zur Zeichnungsebene. Insbesondere kann die Kantenlänge des Sechsecks das 4 bis 6-fache, bevorzugt das 4 bis 5-fache der Blechdicke betragen.

Die Stegbreite in der Zeichnungsebene beträgt bevorzugt das 1 bis 4-fache der Blechdicke des Gitters, besonders bevorzugt das 1 bis 2-fache.

Die Aufnahmen 32 für die Knochenschrauben sind sehr eng über das gesamte Gitter verteilt. Je nach Bedarf kann der Chirurg die Knochenschrauben setzen. Der Innendurchmesser der Aufnahmen 32, die durch nicht vollständig geschlossene Ringe gebildet sind, läßt sich durch den maximalen Durchmesser von Kugeln definieren, die gerade durch die Aufnahmen 32 fallen. In diesem Sinne beträgt der Innendurchmesser der Aufnahmen 32 nicht mehr als 1 mm, insbesondere nicht mehr als 0,8 mm, besonders bevorzugt nicht mehr als 0,4 oder 0,6 mm.

Bevorzugte Blechdicken sind in abhängigen Ansprüchen angegeben.

Das anhand von Fig. 3 beschriebene Gitter ermöglicht eine extreme Dehnung, wobei selbst im stark deformierten Zustand die Abmessungen der Perforationen (Ausnehmungen 32) weitgehend unverfälscht bleiben. Die beschriebene Struktur ermöglicht eine weitgehende Einstellung der Steifigkeit des Gitters, je nach Wahl der Blechstärke und der Stegbreite.

## Patentansprüche

1. Gitter für die Fixierung von Knochenteilen oder für die Überbrückung von Knochenfehlstellen, insbesondere im Schädelund Kieferbereich, aus biokompatiblem Material,
**dadurch gekennzeichnet,**
**daß** an den Gitterpunkten (14) des Gitters (10) gekrümmte Gitter-Stege (16; 36) zusammenlaufen.

2. Gitter nach Anspruch 1,
**dadurch gekennzeichnet,**
**daß** die Stege zwischen jeweils zwei Gitterpunkten (14) S-förmig gekrümmt sind.

3. Gitter nach einem der vorhergehenden Ansprüche,
**dadurch gekennzeichnet,**
**daß** die Stege zwischen zwei Gitterpunkten zick-zack-förmig gekrümmt sind.

4. Gitter nach einem der vorhergehenden Ansprüche,
**dadurch gekennzeichnet,**
**daß** alle Stege zwischen allen Gitterpunkten in zumindest zwei entgegengesetzte Richtungen (R₁, R₂) gekrümmt sind.

5. Gitter nach einem der vorhergehenden Ansprüche,
**dadurch gekennzeichnet,**
**daß** ein in die Ausnehmungen (12) des Gitters gelegter, die Stege gerade berührender Kreis (18) einen Durchmesser (D) kleiner als 2 mm.

6. Gitter nach einem der vorhergehenden Ansprüche,
**dadurch gekennzeichnet,**
**daß** die mittlere Abmessung einer Ausnehmung (12) des Gitters im Bereich von 90% bis 150% der Dicke des Gittermaterials in Richtung senkrecht zur Gitterebene liegt.

7. Gitter nach einem der vorhergehenden Ansprüche,
**dadurch gekennzeichnet,**
**daß** die Gitter-Ausnehmungen (12) zur Aufnahme von Knochenschrauben dienen.

8. Gitter nach einem der vorhergehenden Ansprüche,
**dadurch gekennzeichnet,**
**daß** das Gitter (30) keine geschlossenen kreisförmigen Ringe zur Aufnahme von Knochenschrauben aufweist.

9. Gitter nach einem der vorhergehenden Ansprüche,
**dadurch gekennzeichnet,**
**daß** die Stege (36) nicht vollständig geschlossene Ringe zur Aufnahme von Knochenschrauben bilden.

10. Gitter nach einem der vorhergehenden Ansprüche,
**gekennzeichnet durch**,
mäanderförmige, durchgehend runde Stegreihen.

11. Gitter nach einem der vorhergehenden Ansprüche,
**gekennzeichnet durch**,
mäanderförmige Stegreihen (36) aus halbkreisförmigen Abschnitten.

12. Gitter nach einem der vorhergehenden Ansprüche,
**dadurch gekennzeichnet,**
**daß** die an eine Ausnehmung grenzenden Gitterpunkte (34) ein regelmäßiges Sechseck bilden.

13. Gitter nach Anspruch 12,
**dadurch gekennzeichnet,**
**daß** die Stege zwischen den Eckpunkten des Sechsecks vom Mittelpunkt der Ausnehmung aus gesehen abwechselnd nach außen und innen gewölbt sind, insbesondere halbkreisförmig.

14. Gitter nach einem der Ansprüche 12 oder 13,
**dadurch gekennzeichnet,**
**daß** die Kantenlänge des Sechsecks das 4 bis 6-fache der Blechdicke des Gitters (30) ist.

15. Gitter nach einem der vorhergehenden Ansprüche,
**dadurch gekennzeichnet,**
**daß** die Stegbreite an allen Gitterstellen im wesentlichen gleich ist.

16. Gitter nach einem der vorhergehenden Ansprüche,
**dadurch gekennzeichnet,**
**daß** die Stegbreite das 1 bis 2-fache der Blechdicke des Gitters (30) ist.

17. Gitter nach einem der vorhergehenden Ansprüche,
**dadurch gekennzeichnet,**
**daß** der Innendurchmesser von nicht vollständig geschlossenen Ringen für die Aufnahme von Knochenschrauben kleiner als 1 mm ist.

18. Gitter nach einem der vorhergehenden Ansprüche,
**dadurch gekennzeichnet,**
**daß** es aus Titan besteht.

19. Gitter nach einem der vorhergehenden Ansprüche,
**dadurch gekennzeichnet,**
**daß** es geätzt ist.

20. Gitter nach einem der vorhergehenden Ansprüche,
**dadurch gekennzeichnet,**
**daß** die Dicke des Gittermaterials in Richtung senkrecht zur Gitterebene kleiner als 0,5 mm oder kleiner als 0,2 mm oder kleiner als 0,15 mm oder kleiner als 0,1 mm ist.

21. Gitter nach einem der vorhergehenden Ansprüche,
**dadurch gekennzeichnet,**
**daß** aneinander anschließende, im wesentlichen in die gleiche Richtungen verlaufende Stege (16, 16', 16'', 16''') des Gitters (10) als eine durchgehende Wellenlinie geformt sind.

## Claims

1. A mesh for locating bone parts in position or for bridging bone voids, in particular in the cranial and jaw area, made from biocompatible material,
**characterised in that**
curved mesh webs (16; 36) meet in the mesh points (14) of the mesh (10).

2. The mesh according to Claim 1,
**characterised in that**
the webs between two mesh points (14) each are curved in the shape of an S.

3. The mesh according to one of the previous claims,
**characterised in that**
the webs between two mesh points are curved in a zig-zag shape.

4. The mesh according to one of the previous claims,
**characterised in that**
all webs between all mesh points are curved in at least two opposite directions (R₁, R₂).

5. The mesh according to one of the previous claims,
**characterised in that**
a circle (18) inscribed in the recesses (12) of the mesh and just contacting the webs has a diameter (D) smaller than 2 mm.

6. The mesh according to one of the previous claims,
**characterised in that**
the mean dimension of a recess (12) of the mesh is in the range from 90% to 150% of the thickness of the mesh material in a direction perpendicular to the plane of the mesh.

7. The mesh according to one of the previous claims,
**characterised in that**
the mesh recesses (12) serve to accommodate bone-screws.

8. The mesh according to one of the previous claims,
**characterised in that**
the mesh (30) does not comprise closed circular rings for the accommodation of bone-screws.

9. The mesh according to one of the previous claims,
**characterised in that**
the webs (36) form rings not completely closed for the accommodation of bone-screws.

10. The mesh according to one of the previous claims,
**characterised by**
meander-shaped continuously round web rows.

11. The mesh according to one of the previous claims,
**characterised by**
meander-shaped web rows (36) of semi-circular sections.

12. The mesh according to one of the previous claims,
**characterised in that**
the mesh points (34) bordering a recess form a regular hexagon.

13. The mesh according to Claim 12,
**characterised in that**
that the webs between the corner points of the hexagon, when viewed from the centre of the recess, are alternately arched inwardly and outwardly, in particular semi-circularly.

14. The mesh according to one of Claims 12 or 13,
**characterised in that**
the edge length of the hexagon amounts to 4 to 6 times the sheet metal thickness of the mesh (30).

15. The mesh according to one of the previous claims,
**characterised in that**
the width of the webs at all mesh sites is essentially the same.

16. The mesh according to one of the previous claims,
**characterised in that**
the width of the webs amounts to 1 to 2 times the sheet metal thickness of the mesh (30).

17. The mesh according to one of the previous claims,
**characterised in that**
the inner diameter of not completely closed rings for the accommodation of bone-screws is smaller than 1 mm.

18. The mesh according to one of the previous claims,
**characterised in that**
it consists of titanium.

19. The mesh according to one of the previous claims,
**characterised in that**
it is etched.

20. The mesh according to one of the previous claims,
**characterised in that**
the thickness of the mesh material in a direction perpendicular to the plane of the mesh is smaller than 0.5 mm, or smaller than 0.2 mm, or smaller than 0.15 mm, or smaller than 0.1 mm.

21. The mesh according to one of the previous claims,
**characterised in that**
consecutive webs (16, 6', 16", 16''') of the mesh (10) extending essentially in the same direction are formed as a continuous wavy line.

## Revendications

1. Grille pour la fixation d'éléments d'os ou pour combler des points de défaut osseux, notamment dans la zone du crane et de la mâchoire, en un matériau biocompatible, **caractérisée en ce que** des barres (16; 36) cintrées de la grille convergent au niveau des points (14) de la grille (10).

2. Grille selon la revendication 1, **caractérisée en ce que** les barreaux sont cintrés en forme de S entre deux points respectifs (14) de la grille.

3. Grille selon l'une des revendications précédentes, **caractérisée en ce que** les barreaux entre deux points de la grille sont cintrés avec une forme en zigzag.

4. Grille selon l'une des revendications précédentes, **caractérisée en ce que** tous les barreaux entre tous les points de la grille sont cintrés dans au moins deux directions opposées (R₁, R₂).

5. Grille selon l'une des revendications précédentes, **caractérisée en ce qu'**un cercle (18), qui est disposé dans les ouvertures (12) de la grille et est en contact précisément avec les barreaux, possède un diamètre (D) inférieur à 2 mm.

6. Grille selon l'une des revendications précédentes, **caractérisée en ce que** la dimension moyenne d'une ouverture (12) de la grille se situe dans la gamme de 90 % à 150 % de l'épaisseur du matériau de la grille dans une direction perpendiculaire au plan de la grille.

7. Grille selon l'une des revendications précédentes, **caractérisée en ce que** les ouvertures (12) de la grille servent à loger des vis à os.

8. Grille selon l'une des revendications précédentes, **caractérisée en ce que** la grille (30) ne comporte aucun anneau de forme circulaire fermé pour loger les vis à os.

9. Grille selon l'une des revendications précédentes, **caractérisée en ce que** les barreaux (36) forment des anneaux non complètement fermés servant à loger des vis à os.

10. Grille selon l'une des revendications précédentes, **caractérisée par** des rangées de barreaux de forme sinueuse, entièrement circulaire.

11. Grille selon l'une des revendications précédentes, **caractérisée par** des rangées sinueuses (36) de barreaux, formés de sections semi-circulaires.

12. Grille selon l'une des revendications précédentes, **caractérisée en ce que** les points (34) de la grille, qui délimitent une ouverture, forment un hexagone régulier.

13. Grille selon la revendication 12, **caractérisée en ce que** les barreaux sont cintrés alternativement vers l'extérieur et vers l'intérieur, lorsqu'on regarde à partir du centre de l'ouverture, entre les sommets de l'hexagone et notamment possèdent une forme semi-circulaire.

14. Grille selon l'une des revendications 12 ou 13, **caractérisée en ce que** la longueur des côtés de l'hexagone est comprise entre 4 et 6 fois l'épaisseur de la tôle de la grille (30).

15. Grille selon l'une des revendications précédentes, **caractérisée en ce que** la largeur des barrettes est essentiellement la même en tous les points de la grille.

16. Grille selon l'une des revendications précédentes, **caractérisée en ce que** la largeur de la grille est comprise entre 1 et 2 fois l'épaisseur de la tôle de la grille (30).

17. Grille selon l'une des revendications précédentes, **caractérisée en ce que** le diamètre intérieur d'anneaux non complètement fermés, servant à recevoir des vis à os, est inférieur à 1 mm.

18. Grille selon l'une des revendications précédentes, **caractérisée en ce qu'**elle est formée de titane.

19. Grille selon l'une des revendications précédentes, **caractérisée en ce qu'**elle est formée par attaque chimique.

20. Grille selon l'une des revendications précédentes, **caractérisée en ce que** l'épaisseur du matériau de la grille dans une direction perpendiculaire au plan de la grille est inférieure à 0,5 m ou est inférieure à 0,2 mm ou est inférieure à 0,15 mm ou est inférieure à 0,1 mm.

21. Grille selon l'une des revendications précédentes, **caractérisée en ce que** les barreaux (16, 16', 16", 16"'), qui se raccordent entre eux et s'étendent essentiellement dans la même direction, de la grille (10) sont agencés sous la forme d'une ligne ondulée continue.
